# FASCICULE DE BREVET EUROPEEN

(11) **EP 1 729 785 B1**
(45) Date de publication et mention de la délivrance du brevet: **07.05.2008**
(21) Numéro de dépôt: 05730830.6
(22) Date de dépôt: 18.02.2005
(51) Int. Cl.: A61K 33/00, A61P 27/06

(54) **COLLYRE CONSTITUE D"UNE EAU ARGILEUSE FILTREE UTILISE POUR LE TRAITEMENT DU GLAUCOME**
AUGENTROPFEN MIT GEFILTERTEM LEHMHALTIGEN WASSER ZUR BEHANDLUNG VON GLAUKOM
EYE DROPS CONSISTING OF FILTERED CLAYEY WATER FOR TREATING GLAUCOMA

(30) Priorité: 09.03.2004 FR 0402464
(43) Date de publication de la demande: 13.12.2006
(73) Titulaire: Neumann, Armand, F-75019 Paris (FR)
(72) Inventeur: Neumann, Armand, F-75019 Paris (FR)
(86) Numéro de dépôt international: PCT/FR2005/000393
(87) Numéro de publication internationale: WO 2005/097143

(56) Documents cités:
- EP-A- 0 116 240
- WO-A-03/059263
- CA-A- 2 093 573
- V. SABINI: "espace pro" LABO-HEVEA SITE, LABORATOIRES LBVH, [Online] 2000, - 2004 XP002300065 GENEVE Extrait de l'Internet: URL:http://www.labo-hevea.com/fiche_produi t.php?langue=FR&id=MON011> [extrait le 2004-10-11]
- ANONYMOUS: "CureZone Newsletter" INTERNET ARTICLE, [Online] XP002330326 Extrait de l'Internet: URL:http://www.curezone.com/forums/m.asp?f =143&i=9> [extrait le 2005-06-01]
- PHILIT M.: "La gestion du parasitisme" LE JURA, AGRICOLE ET RURAL, [Online] 2 mai 2005 (2005-05-02), XP002330327 Extrait de l'Internet: URL:http://www.juragricole.com/news/archiv estory.php/aid/4852/La_gestion_du_parasiti sme.html> [extrait le 2005-06-01]
- ANONYMOUS: "Minéraux industriels" INTERNET ARTICLE, [Online] XP002330328 Extrait de l'Internet: URL:http://www.mrn.gouv.qc.ca/gaspesie-ile s-de-la-madeleine/mines/mines-potentiel-mi neraux.jsp> [extrait le 2005-06-01]

## Description

### INVENTION :

La présente invention concerne un collyre constitué d'une eau argileuse filtrée et purifiée de toutes ses particules, utilisé pour le traitement du Glaucome.

### PROCEDE DE FABRICATION :

Ce collyre est obtenu à partir d'un mélange, dans un récipient en verre épais, d'un quart de poudre d'argile verte très fine et de trois quarts d'eau froide ordinaire. Couvrir hermétiquement le récipient, ne jamais utiliser d'objet métallique et laisser déposer au moins une semaine avant de filtrer longuement cette eau en prenant soin de ne pas remuer le limon déposé au fond, afin d'éliminer toutes les particules et ainsi obtenir une eau parfaitement purifiée.
Ce collyre est prêt à l'usage.

### INDICATIONS THERAPEUTIQUES DU PRODUIT :

Ce collyre est destiné au traitement du Glaucome afin d'abaisser l'hypertension jusqu'à rendre normale la tension intra oculaire.

Il facilite l'évacuation de l'humeur aqueuse qui, lorsqu'elle est en trop-plein, exerce une pression fâcheuse, voire dangereuse sur le nerf optique.

Autre avantage : étant donné que cette eau argileuse ne comporte pas de contre-indication, rien ne s'oppose à plusieurs instillations (3,4 instillations par 24 heures) si besoin est.

### PROPRIETES :

Les propriétés de cette eau argileuse sont essentiellement sédatives, antiseptiques, toniques ainsi qu'une radioactivité déterminante quant à l'équilibre des fonctions vitales de l'oeil. Ce qui lui confère une efficacité qui, à l'usage s'avère indéniable, et cela sans aucune contre-indication.

Certes les vertus de l'argile sont bien connues, mais l'eau argileuse n'a jamais été utilisée en collyre.

Mais encore fallait-il oser l'instiller dans un oeil malade et de surcroît quand on est borgne. Et dès les premiers jours j'en ai ressenti les bienfaits. J'étais sur la bonne voie, avec la confirmation sur le long terme.
Et dernier point non négligeable, compte tenu de la simplicité de ce produit naturel, son prix de revient serait de beaucoup inférieur à tous ceux actuellement sur le marché.

## Revendications

1. Utilisation d'une eau argileuse filtrée et purifiée de ses particules pour préparer un collyre destiné au traitement du Glaucome afin d'abaisser l'hypertension jusqu'à rendre normale la tension intra-oculaire.

## Claims

1. Use of clayey water filtered and purified from all its particles in order to prepare eye drops used for treating Glaucoma and decline the hypertension until restoring a normal intraocular pressure.

## Patentansprüche

1. Anwendung eines tonhaltigen Wassers, aus dem die Partikel herausgefiltert und das gereinigt wurde, zwecks Vorbereitung eines Augenwassers, das zur Glaukom-Behandlung bestimmt ist, mit der Zielsetzung, die Hypertonie zu vermindern, damit schließlich ein normaler Augeninnendruck eintritt.
